# EUROPEAN PATENT APPLICATION

(11) **EP 4 813 308 A1**
(43) Date of publication of application: **30.09.2026**
(21) Application number: 26168709.9
(22) Date of filing: 27.03.2026
(51) Int. Cl.: A61B 6/04

(54) **MEDICAL EXAMINATION TABLE AND MEDICAL IMAGE DIAGNOSTIC APPARATUS**

(30) Priority: 28.03.2025 JP 2025056483
(71) Applicant: FUJIFILM Corporation, Tokyo 106-8620 (JP)
(72) Inventor: IWASAWA, Kohjiro, Tokyo, 1068620 (JP); OTAKE, Yosuke, Tokyo, 1068620 (JP); ARAI, Koichi, Tokyo, 1068620 (JP)
(74) Representative: Dehns Germany Partnerschaft mbB

(57) **Abstract**

A medical examination table (20) includes a fixing belt (31) and an elevating mechanism. The fixing belt (31) includes a first belt extending from a first side portion that is a side portion on one side of a placement portion in the lateral direction, and, a second belt extending from a second side portion that is a side portion on the other side of the placement portion, and fixes the subject on the placement portion by joining the first belt and the second belt. The elevating mechanism is provided at a first side portion side of the placement portion, and includes a first connecting portion to which the first belt is connected, and a second connecting portion (43L) to which the second belt is connected, and elevates a part of the first belt when pulling the second belt and lowers the part of the first belt when loosening the second belt.

## Description

### BACKGROUND OF THE INVENTION

### 1. Field of the Invention

The present invention relates to a medical examination table and a medical image diagnostic apparatus, and particularly to a medical examination table and a medical image diagnostic apparatus comprising a fixing band that fixes a subject.

### 2. Description of the Related Art

Medical image diagnostic apparatuses, such as a magnetic resonance imaging (MRI) apparatus, an X-ray diagnostic apparatus, an X-ray computed tomography (CT) apparatus, a positron emission tomography (PET) apparatus, and a single photon emission computed tomography (SPECT) apparatus, are known as a device that, for the purpose of medical diagnosis, irradiates an organ or a cell with energy from an outside to measure a reflection amount or a transmission amount thereof, and visualizes biological information such as a form or a function of a target. These medical image diagnostic apparatuses generally include an examination table (medical examination table), and the subject (patient) is placed on the examination table to perform imaging.

In imaging in the medical image diagnostic apparatus, the subject needs to maintain a stationary state during imaging. On the other hand, imaging may take a long time. Therefore, in the medical image diagnostic apparatus, the subject may be fixed to the examination table to perform imaging. In this case, an examination table comprising a fixing tool is used. A belt is generally used as the fixing tool (for example, see JP2013-198580A, JP1991-053207U (JP-H03-053207U), and JP2008-099730A).

### SUMMARY OF THE INVENTION

The belt has flexibility and is in a state of hanging down from both sides of a top plate portion of the examination table in an unused state. Therefore, in a case in which the subject is fixed by the belt, the belt in the state of hanging down needs to be pulled up from both sides of the examination table. In a case in which one technician performs a belt installation operation, first, the technician needs to pull up one belt on one side of the examination table, and then move to the opposite side of the examination table to pull up the other belt. Therefore, there is a problem that the installation takes time and effort. In addition, in a case of the MRI apparatus, a receive coil may be fixed to the subject by the belt. In this case, there is a problem that the receive coil may fall or be displaced during a fixing operation. The technician can also pull up the belt on the opposite side by leaning over the subject on the examination table, but in this case, the technician may need to be in close contact with the subject, which may cause discomfort to both of the technician and the subject.

The present invention has been made in view of such circumstances, and an object of the present invention is to provide a medical examination table and a medical image diagnostic apparatus that can easily fix a subject.
[1] A medical examination table comprising: a placement portion on which a subject is placed; a fixing belt that includes a first belt portion extending in a lateral direction of the placement portion from a first side portion that is a side portion on one side of the placement portion in the lateral direction, and a second belt portion extending in the lateral direction of the placement portion from a second side portion that is a side portion on the other side of the placement portion in the lateral direction of the placement portion, and that fixes the subject placed on the placement portion by joining the first belt portion and the second belt portion; and an elevating mechanism that is provided on a first side portion side of the placement portion, that includes a first connecting portion to which the first belt portion is connected, and a second connecting portion to which the second belt portion is connected, and that elevates at least a part of the first belt portion by pulling operation of the second belt portion and lowers the at least part of the first belt portion by loosening operation of the second belt portion.
[2] The medical examination table according to [1], in which the elevating mechanism includes a rotating member that rotates about an axis extending in a longitudinal direction of the placement portion, the first connecting portion and the second connecting portion are provided in the rotating member, and the rotating member is rotated by the pulling operation and the loosening operation of the second belt portion to elevate and lower the at least part of the first belt portion.
[3] The medical examination table according to [2], in which the rotating member includes a shaft portion, a first arm portion that extends in a radial direction from the shaft portion, and a second arm portion that extends in a direction intersecting the first arm portion from the shaft portion or the first arm portion, the first connecting portion is provided in the first arm portion, and the second connecting portion is provided in the second arm portion.
[4] The medical examination table according to [3], in which the first connecting portion is provided on a distal end portion of the first arm portion, the second connecting portion is provided on a distal end portion of the second arm portion, and a length of the first arm portion is longer than a length of the second arm portion.
[5] The medical examination table according to [2], in which the rotating member includes a shaft portion, a first arm portion that extends in a radial direction from the shaft portion, and a second arm portion that extends in a direction opposite to the first arm portion from the shaft portion, the first connecting portion is provided in the first arm portion, the second connecting portion is provided in the second arm portion, and the elevating mechanism further includes a guide member that guides the second belt portion in a direction in which the rotating member rotates in a case in which the second belt portion is pulled.
[6] The medical examination table according to [2], in which the rotating member includes a shaft portion and an arm portion that extends in a radial direction from the shaft portion, the first connecting portion is provided on a distal end side of the arm portion, and the second connecting portion is provided on a base end side of the arm portion.
[7] The medical examination table according to [2], in which the first belt portion has a higher stiffness than the second belt portion.
[8] The medical examination table according to [2], in which the first belt portion has a portion of which flexibility changes in conjunction with the pulling operation and the loosening operation of the second belt portion at least at a base end portion, and the portion is configured by connecting a plurality of members in an extending direction of the first belt portion, is rigid by joining the members adjacent to each other by the pulling operation of the second belt portion and is flexible by separating the members adjacent to each other by the loosening operation of the second belt portion.
[9] The medical examination table according to any one of [1] to [8], in which the fixing belt further includes a third belt portion that branches from the second belt portion, that extends from the second side portion of the placement portion in the lateral direction of the placement portion together with the second belt portion, and that is joinable to the first belt portion and/or the second belt portion.
[10] The medical examination table according to [9], further comprising: an engagement portion that is provided on a second side portion side of the placement portion and on which the third belt portion is hooked and engaged.
[11] The medical examination table according to [9] or [10], in which the second belt portion is joined to the first belt portion via a hook-shaped retainer, and the third belt portion is joined to the first belt portion and/or the second belt portion via a hook-and-loop fastener.
[12] The medical examination table according to any one of [1] to [11], in which the elevating mechanism is movable in a longitudinal direction of the placement portion.
[13] The medical examination table according to any one of [2] to [8], in which the elevating mechanism further includes a restricting member that restricts a rotation range of the rotating member.
[14] The medical examination table according to any one of [1] to [13], in which the placement portion has a space through which the second belt portion passes from the first side portion side to a second side portion side.
[15] The medical examination table according to [14], in which the placement portion includes a top plate, a mattress provided on the top plate, and the space is provided on the top plate or the mattress or between the top plate and the mattress.
[16] A medical image diagnostic apparatus comprising: the medical examination table according to any one of [1] to [15], in which the medical image diagnostic apparatus captures a medical image of the subject placed on the medical examination table.

According to the present invention, the subject can be easily fixed.

### BRIEF DESCRIPTION OF THE DRAWINGS

FIG. 1 is a perspective view showing an example of an MRI apparatus.
FIG. 2 is a perspective view showing a schematic configuration of an examination table.
FIG. 3 is a plan view of an installation portion of a fixing tool of a top plate.
FIG. 4 is a cross-sectional view taken along line 4-4 of FIG. 3.
FIG. 5 is a perspective view showing a schematic configuration of a belt elevating function.
FIG. 6 is an explanatory view of a method of fixing a subject using a fixing tool.
FIG. 7 is an explanatory view of a method of fixing a subject using the fixing tool.
FIG. 8 is an explanatory view of a method of fixing a subject using the fixing tool.
FIG. 9 is a view showing a first modification example of a belt elevating mechanism.
FIG. 10 is a view showing the first modification example of the belt elevating mechanism.
FIG. 11 is a view showing a second modification example of the belt elevating mechanism.
FIG. 12 is a view showing the second modification example of the belt elevating mechanism.
FIG. 13 is a view showing a third modification example of the belt elevating mechanism.
FIG. 14 is a view showing the third modification example of the belt elevating mechanism.
FIG. 15 is a view showing a fourth modification example of the belt elevating mechanism.
FIG. 16 is a view showing the fourth modification example of the belt elevating mechanism.
FIG. 17 is a view showing the fourth modification example of the belt elevating mechanism.
FIG. 18 is a plan view of a second embodiment of the fixing tool.
FIG. 19 is a cross-sectional view taken along a line 19-19 of FIG. 18.
FIG. 20 is an explanatory view of a method of fixing a subject using a fixing tool.
FIG. 21 is an explanatory view of a method for fixing a subject using a fixing tool.
FIG. 22 is a plan view of a third embodiment of the fixing tool.
FIG. 23 is a cross-sectional view taken along a line 23-23 of FIG. 22.

### DESCRIPTION OF THE PREFERRED EMBODIMENTS

Hereinafter, preferred embodiments of the present invention will be described with reference to the accompanying drawings.

### First Embodiment

Here, a case where the present invention is applied to an MRI apparatus will be described as an example. The MRI apparatus is an apparatus that excites nuclear spins of a subject placed in a static magnetic field with a radio frequency (RF) signal (high-frequency signal) at a Larmor frequency and reconstructs a magnetic resonance (MR) signal generated from the subject in response to the excitation to generate an image (medical image). In the present embodiment, the MRI apparatus is an example of a medical image diagnostic apparatus.

### Overall Configuration of MRI Apparatus

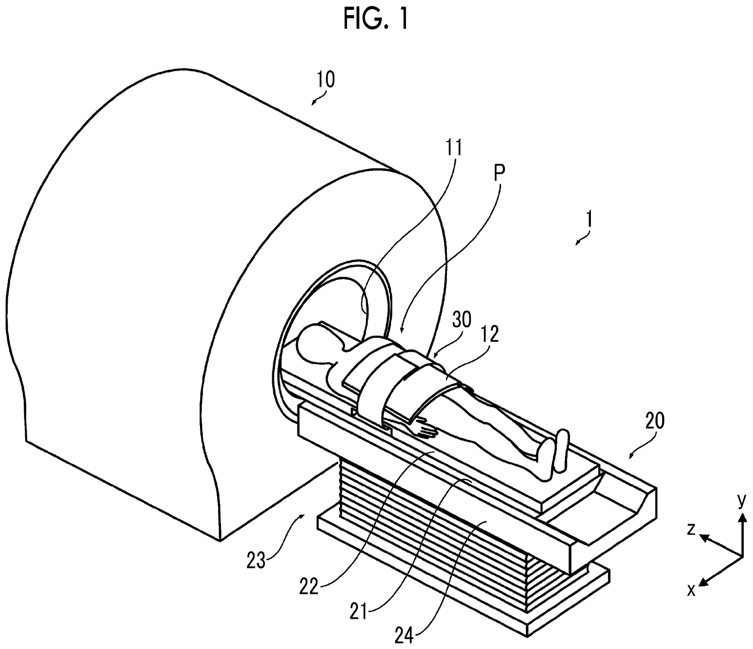

FIG. 1 is a perspective view showing an example of an MRI apparatus. In FIG. 1, an x-axis, a y-axis, and a z-axis are three axes orthogonal to each other. A y-axis direction is defined as a vertical direction (up-down direction), and a plane formed by the x-axis and the z-axis is defined as a horizontal plane.

An MRI apparatus 1 shown in FIG. 1 is a so-called tunnel type MRI apparatus, and includes a magnet 10 that generates a static magnetic field and an examination table 20. The examination table 20 is an example of a medical examination table.

### Magnet

The magnet 10 includes an imaging space (examination space) 11. As an example, in the present embodiment, the magnet 10 includes the imaging space 11 having a cylindrical shape (has a so-called tunnel type structure). The magnet 10 can also adopt a so-called open type structure. A subject P is inserted into the imaging space 11 and is imaged (examined).

The magnet 10 includes a gradient magnetic field coil (not shown) that generates a gradient magnetic field in a housing and a transmit coil (not shown) that transmits an RF pulse.

The MRI apparatus 1 further includes a receive coil 12 that receives the MR signal generated from the subject P. The receive coil 12 is disposed at an imaging target portion (examination target portion) of the subject P. FIG. 1 shows an example in a case in which the imaging target portion is an abdomen. An image (tomographic image of the subject P) is generated by reconstructing the MR signal received by the receive coil 12.

### Examination Table

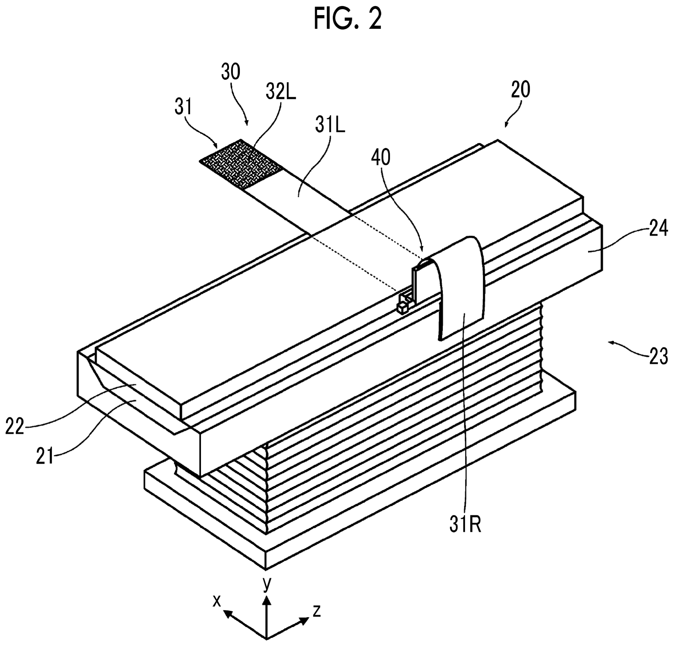

FIG. 2 is a perspective view showing a schematic configuration of the examination table.

The examination table 20 includes a top plate 21 on which the subject P is placed, a mattress 22 disposed on the top plate 21, a base 23 that supports the top plate 21, a fixing tool 30 that fixes the subject P placed on the top plate 21, and the like.

The top plate 21 has a rectangular flat plate shape in a plan view. A direction along a long side of the top plate 21 (z-axis direction of FIG. 2) is defined as a longitudinal direction, and a direction along a short side of the top plate 21 (x-axis direction of FIG. 2) is defined as a lateral direction. In addition, a height direction of the top plate 21 (y-axis direction of FIG. 2) is defined as a thickness direction. It should be noted that the longitudinal direction is synonymous with a front-rear direction, and the lateral direction is synonymous with a left-right direction and a width direction. In addition, the thickness direction is synonymous with an up-down direction.

The mattress 22 is disposed on an upper surface of the top plate 21 as a cushion. The mattress 22 has a rectangular flat plate shape in a plan view and is disposed along the longitudinal direction of the top plate 21. The mattress 22 has a lateral width narrower than a lateral width (width in the lateral direction) of an upper surface portion of the top plate 21 and is disposed at a center of the upper surface of the top plate 21 in the width direction. Therefore, the upper surface of the top plate 21 includes a region that is not covered with the mattress 22 along both edges.

The subject P is placed on the top plate 21 with the mattress 22 (see FIG. 1). In general, the subject P is placed in a supine posture along the longitudinal direction of the top plate 21. Therefore, the subject P is placed such that a body axis is along the longitudinal direction of the top plate 21. In the present embodiment, a combination of the top plate 21 and the mattress 22 constitutes a placement portion of the subject.

The base 23 includes a top-plate support portion 24 that supports the top plate 21. The top plate 21 is supported by the top-plate support portion 24 and is horizontally disposed. The base 23 includes a mechanism that elevates the top plate 21 in the vertical direction and a mechanism that horizontally slides the top plate 21 along the longitudinal direction. These mechanisms are known mechanisms in a medical examination table. Therefore, a detailed description thereof will be omitted. The base 23 may further include a mechanism that moves the top plate 21 along the lateral direction.

The top plate 21 is inserted into the imaging space 11 by horizontally sliding the top plate 21 along the longitudinal direction. In addition, as a result, the subject P on the top plate 21 is inserted into the imaging space 11. A direction in which the top plate 21 is inserted into the imaging space 11 (direction in which the top plate 21 moves toward the imaging space 11) is defined as an insertion direction, and a direction in which the top plate 21 retract from the imaging space 11 (direction in which the top plate 21 moves away from the imaging space 11) is defined as a retreat direction.

### Fixing Tool

The fixing tool 30 fixes the subject P placed on the top plate 21.

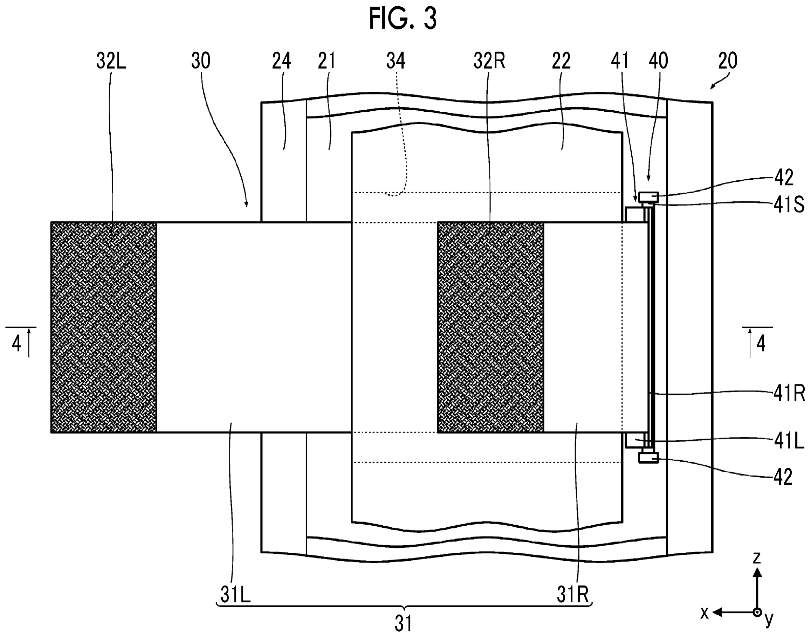

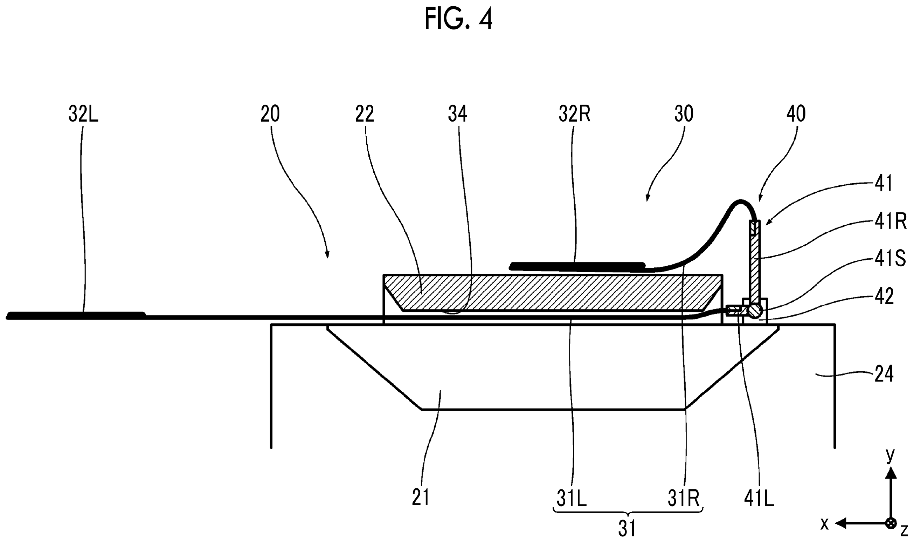

FIG. 3 is a plan view of an installation portion of a fixing tool of a top plate. Further, FIG. 4 is a cross-sectional view taken along line 4-4 of FIG. 3.

As shown in FIGS. 3 and 4, the fixing tool 30 includes a fixing belt 31, and the fixing belt 31 is wound around the subject P to fix the subject P to the top plate 21.

The fixing belt 31 includes a right belt portion 31R extending in the lateral direction of the top plate 21 from a side portion on a right side of the top plate 21 in a traveling direction and a left belt portion 31L extending in the lateral direction of the top plate 21 from a side portion on a left side of the top plate 21 in the traveling direction of the top plate 21. The right belt portion 31R and the left belt portion 31L are connected to each other via a belt elevating mechanism 40 to constitute one elongated fixing belt 31. In the present embodiment, the side portion on the right side of the top plate 21 in the traveling direction is an example of a first side portion, and the side portion on the left side is an example of a second side portion in the traveling direction. In addition, the right belt portion 31R is an example of a first belt portion, and the left belt portion 31L is an example of a second belt portion.

The right belt portion 31R and the left belt portion 31L are both formed of a flexible belt-shaped sheet. In addition, the right belt portion 31R and the left belt portion 31L have hook-and-loop fasteners 32R and 32L at distal end portions thereof, respectively. The hook-and-loop fasteners 32R and 32L are provided on overlapping surfaces of the right belt portion 31R and the left belt portion 31L (mutually facing surfaces of the right belt portion 31R and the left belt portion 31L when the right belt portion 31R and the left belt portion 31L are overlapped) when the right belt portion 31R and the left belt portion 31L are overlapped and joined. In the present embodiment, the left belt portion 31L is overlapped on the right belt portion 31R, and the right belt portion 31R and the left belt portion 31L are joined to each other. Therefore, the hook-and-loop fastener 32R is provided on a surface that is disposed on an outer side (upper side) of the right belt portion 31R in a case in which the right belt portion 31R is wound around the subject P. On the other hand, the hook-and-loop fastener 32L is provided on a surface that is disposed on an inner side (lower side) of the left belt portion 31L in a case in which the left belt portion 31L is wound around the subject P. The hook-and-loop fasteners 32R and 32L have configurations in which one has hook-shaped pile, and the other has densely formed loop-shaped pile. As an example, in the present embodiment, the hook-and-loop fastener 32R on the right belt portion 31R side has a configuration having hook-shaped pile, and the hook-and-loop fastener 32L on the left belt portion 31L side has a configuration having densely formed loop-shaped pile.

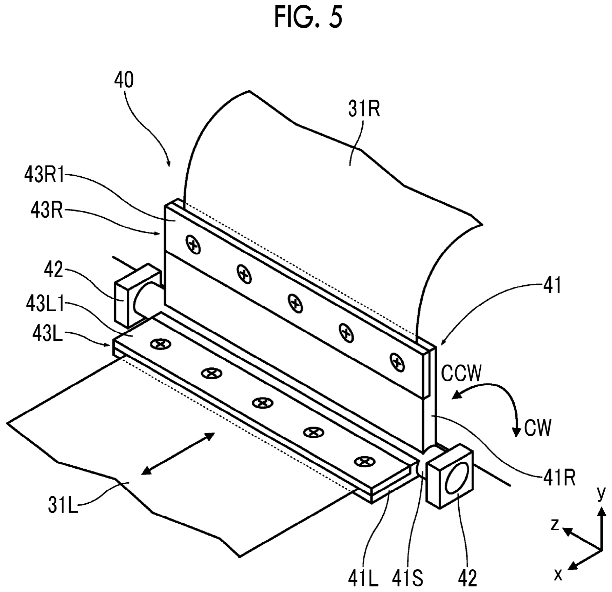

FIG. 5 is a perspective view showing a schematic configuration of a belt elevating function.

The belt elevating mechanism 40 is provided on one side of the top plate 21 in the lateral direction. In the present embodiment, the belt elevating mechanism 40 is disposed at an edge portion of an upper surface of the top plate 21 on the right side in the traveling direction (first side portion side) of the top plate 21 and is disposed in parallel with the mattress 22 (see FIGS. 3 and 4).

The belt elevating mechanism 40 includes a rotating body 41 that rotates about an axis along the longitudinal direction of the top plate 21. The rotating body 41 includes a rotation shaft 41S and a first arm portion 41R and a second arm portion 41L that extend in a radial direction from the rotation shaft 41S. In the present embodiment, the rotating body 41 is an example of a rotating member.

The rotation shaft 41S includes both end portions that are supported by a pair of bearing portions 42 provided in the top plate 21 and is disposed along the longitudinal direction of the top plate 21. In the present embodiment, the rotation shaft 41S is an example of a shaft portion.

The first arm portion 41R and the second arm portion 41L are configured of rectangular plate-shaped members that extend in the radial direction from the rotation shaft 41S and are disposed to intersect each other. As an example, in the present embodiment, the first arm portion 41R and the second arm portion 41L are disposed to be orthogonal to each other. Therefore, in the present embodiment, an integrated body of the first arm portion 41R and the second arm portion 41L has an L-shape in a cross section orthogonal to the rotation shaft 41S.

In the present embodiment, the first arm portion 41R and the second arm portion 41L have different lengths in the radial direction of the rotation shaft 41S. Specifically, the first arm portion 41R has a length longer than a length of the second arm portion 41L.

The first arm portion 41R includes a first connecting portion 43R to which the right belt portion 31R of the fixing belt 31 is connected. The first connecting portion 43R is provided on a distal end of the first arm portion 41R and grips and holds a base end portion of the right belt portion 31R. Specifically, at a distal end portion of the first arm portion 41R, the base end portion of the right belt portion 31R is held by a plate-shaped clamping member 43R1. By holding the base end portion of the right belt portion 31R with the first connecting portion 43R, the right belt portion 31R is connected to and integrated with the first arm portion 41R.

The second arm portion 41L includes a second connecting portion 43L to which the left belt portion 31L of the fixing belt 31 is connected. The second connecting portion 43L is provided at a distal end of the second arm portion 41L and grips and holds a base end portion of the left belt portion 31L. Specifically, at a distal end portion of the second arm portion 41L, the base end portion of the left belt portion 31L is held by a plate-shaped clamping member 43L1. By holding the base end portion of the left belt portion 31L with the second connecting portion 43L, the left belt portion 31L is connected to and integrated with the second arm portion 41L.

The right belt portion 31R is connected to the first connecting portion 43R, and the left belt portion 31L is connected to the second connecting portion 43L, so that the right belt portion 31R and the left belt portion 31L are connected on the same straight line to constitute one elongated fixing belt 31.

In the belt elevating mechanism 40 configured as described above, in a case in which the left belt portion 31L is pulled, the rotating body 41 rotates in a counterclockwise direction CCW as viewed from a rear side of the top plate 21 in the traveling direction. As a result, the first arm portion 41R stands, and the right belt portion 31R is pulled up. More specifically, a base end portion of the right belt portion 31R is pulled up. On the other hand, in a case in which the pulling force on the left belt portion 31L is released, a weight of the right belt portion 31R causes the rotating body 41 to rotate in the clockwise direction CW. As a result, the first arm portion 41R falls, and the right belt portion 31R that has been pulled up is lowered.

In a case in which the rotating body 41 rotates in a direction (counterclockwise direction CCW) in which the first arm portion 41R stands, the second arm portion 41L comes into contact with the upper surface of the top plate 21 at a predetermined position, and the rotation is restricted. In addition, in a case in which the rotating body 41 rotates in a direction (clockwise direction CW) in which the first arm portion 41R falls, the first arm portion 41R comes into contact with the top-plate support portion 24 at a predetermined position, and the rotation is restricted. As a result, the rotating body 41 is held to be rotatable only within a necessary range. As an example, in the present embodiment, the rotating body 41 is held to be rotatable within a range of approximatively 90°. As a result, the first arm portion 41R rotates between a state in which the first arm portion 41R is substantially horizontal and a state in which the first arm portion 41R is substantially vertical. In the present embodiment, the top plate 21 and the top-plate support portion 24 are examples of a restricting member that restricts a rotation range of the rotating body 41.

As shown in FIGS. 3 and 4, the mattress 22 includes a tunnel-shaped space 34 that connects side surfaces on both sides in the lateral direction corresponding to a disposition position of the belt elevating mechanism 40. The space 34 constitutes a passage through which the fixing belt 31 is pulled from one side (first side portion side) of the top plate 21 to the other side (second side portion side). The left belt portion 31L of the fixing belt 31 is pulled out from the right side (first side portion side) of the top plate 21 in the traveling direction to the left side (second side portion side) through the space 34.

### Method of Fixing Subject by Fixing Tool

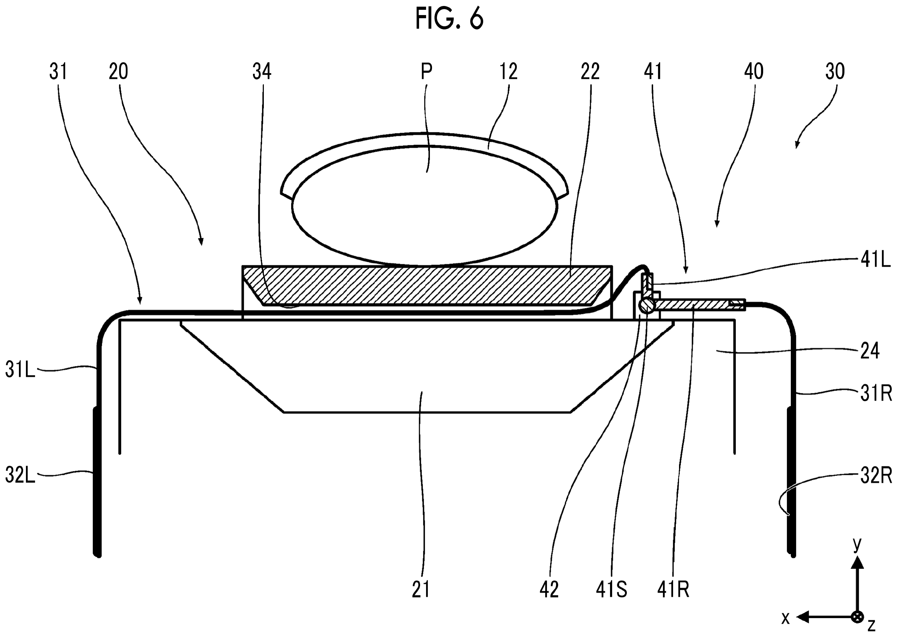

Next, a method of fixing the subject P on the top plate 21 using the fixing tool 30 of the present embodiment will be described. FIGS. 6 to 8 are explanatory views of a method of fixing the subject using the fixing tool.

As shown in FIG. 6, first, the subject P is placed on the mattress 22 of the top plate 21. As described above, the subject P is placed on the top plate 21 in a supine posture along the longitudinal direction of the top plate 21. For convenience, the subject P is shown by an ellipse in FIGS. 6 to 8.

As shown in FIG. 6, in a case in which the subject P is placed on the mattress 22 of the top plate 21, the fixing belt 31 is retracted from the mattress 22. As a result, the fixing belt 31 is held in the state of hanging down on both sides of the examination table 20. More specifically, the right belt portion 31R is held in a state of hanging down on a right side portion of the examination table 20 by its own weight, and the left belt portion 31L is held in the state of hanging down on a left side portion of the examination table 20 by its own weight. In addition, the belt elevating mechanism 40 holds the first arm portion 41R of the rotating body 41 in a state of being substantially horizontal.

After the subject P is placed on the mattress 22 of the top plate 21, the receive coil 12 is set at the imaging target portion of the subject P.

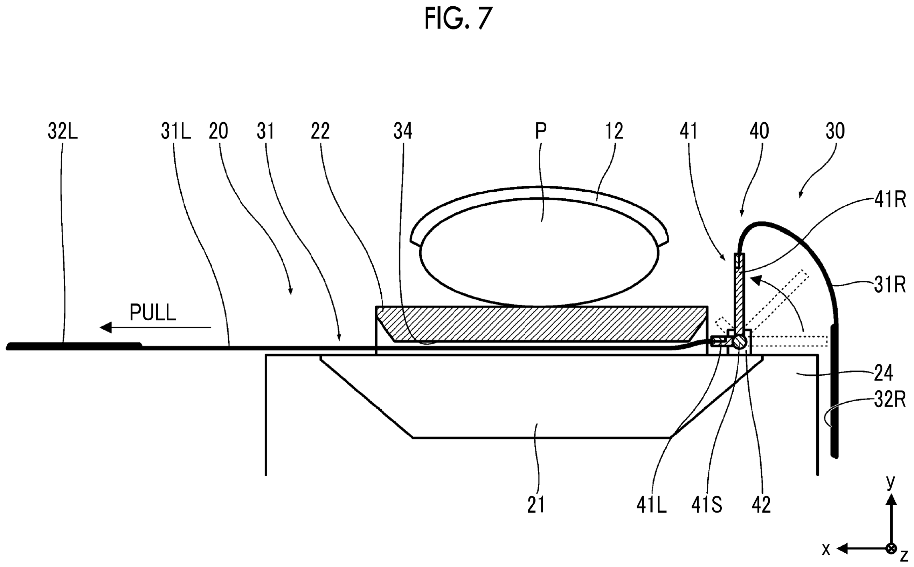

After the receive coil 12 is set, the technician stands at the side portion on the left side of the top plate 21 in the traveling direction and pulls the left belt portion 31L extending from the side portion on the left side of the top plate 21. As a result, as shown in FIG. 7, the second arm portion 41L of the rotating body 41 connected to the left belt portion 31L is pulled toward the side portion on the left side of the top plate 21 in the traveling direction, and the rotating body 41 rotates counterclockwise. Then, the first arm portion 41R stands substantially vertically by the rotating body 41 rotating counterclockwise. As a result, the right belt portion 31R is pulled up.

By pulling up the right belt portion 31R, the right belt portion 31R can be retrieved from the side portion on the left side of the top plate 21 in the traveling direction. That is, the technician can retrieve the belt portion from one side to the other side of the top plate 21 in the lateral direction without moving.

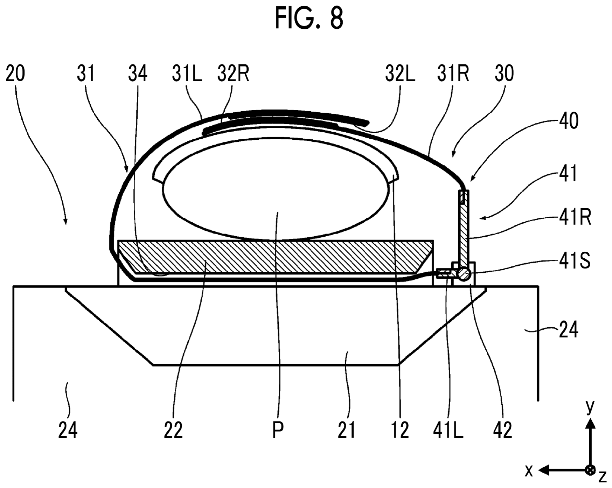

After the right belt portion 31R is retrieved, as shown in FIG. 8, the right belt portion 31R and the left belt portion 31L are wound around the subject P, and the right belt portion 31R and the left belt portion 31L are joined to each other. In the present embodiment, the left belt portion 31L is overlapped on the right belt portion 31R, and the right belt portion 31R and the left belt portion 31L are joined to each other. By overlapping the left belt portion 31L on the right belt portion 31R, the hook-and-loop fastener 32R on the right belt portion 31R side and the hook-and-loop fastener 32L on the left belt portion 31L side are adhered to each other, and the right belt portion 31R and the left belt portion 31L are joined to each other. As a result, the subject P is fixed to the top plate 21.

In a case in which the fixing is released, the hook-and-loop fastener 32L on the left belt portion 31L side is peeled off from the hook-and-loop fastener 32R on the right belt portion 31R side, and the joining of the right belt portion 31R and the left belt portion 31L is released.

The fixing belt 31 in which the joining of the right belt portion 31R and the left belt portion 31L is released is retracted from the top plate 21. By retracting the fixing belt 31 from the top plate 21, the right belt portion 31R and the left belt portion 31L are held in the state of hanging down from both sides of the top plate 21. In this case, in the rotating body 41 of the belt elevating mechanism 40, the first arm portion 41R is pulled by the weight of the right belt portion 31R, and the first arm portion 41R is held in a state of being substantially horizontal as shown in FIG. 6.

As described above, according to the examination table 20 of the present embodiment, in a case in which the subject P is fixed on the top plate 21, the right belt portion 31R on the other side can be pulled up by the pulling operation of the left belt portion 31L. As a result, the fixing operation of the subject P can be facilitated. That is, since the technician can retrieve the belt portion on the other side while being positioned on one side of the examination table 20, the technician can save the effort of moving. As a result, the subject P can be easily and quickly fixed. In a case in which the subject P is fixed by the fixing belt 31, the pulling operation of the belt portion (left belt portion 31L) on one side is always performed. Therefore, the belt portion (right belt portion 31R) on the other side can be pulled up by the operation that is usually performed. As a result, the fixing operation can be efficiently performed. In addition, since the technician does not need to lean over the subject P as closely as possible, it is possible to suppress the discomfort to both the technician and the subject.

### Modification Example

### Modification Example of Belt elevating mechanism

As described above, the belt elevating mechanism 40 of the present embodiment is configured to rotate the rotating body 41 by the pulling operation and the loosening operation of the left belt portion 31L on one side and to elevate and lower the right belt portion 31R on the other side. The configuration of the rotating body and the mechanism that rotates the rotating body are not limited to the above configuration. Hereinafter, a modification example of the belt elevating mechanism will be described.

### First Modification Example

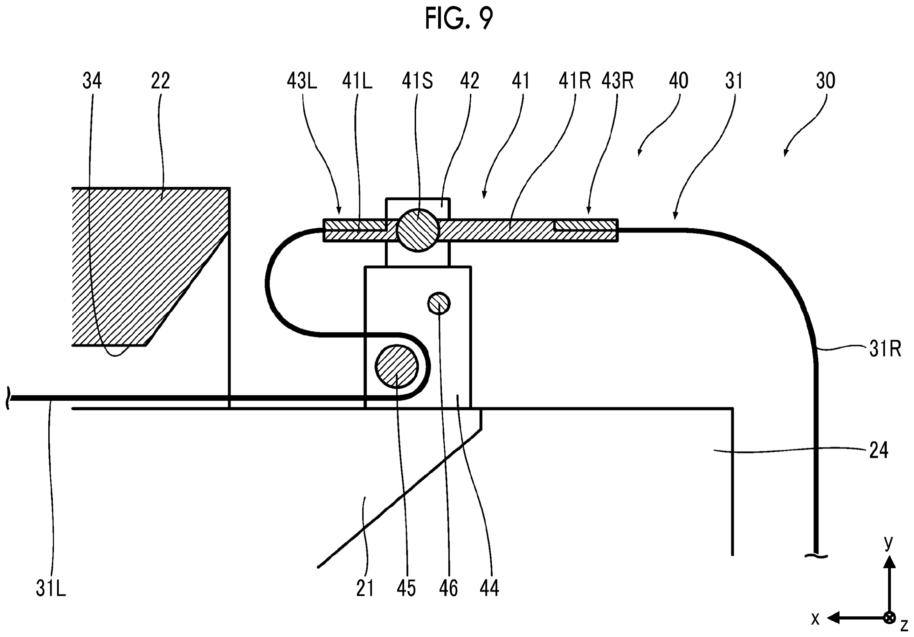

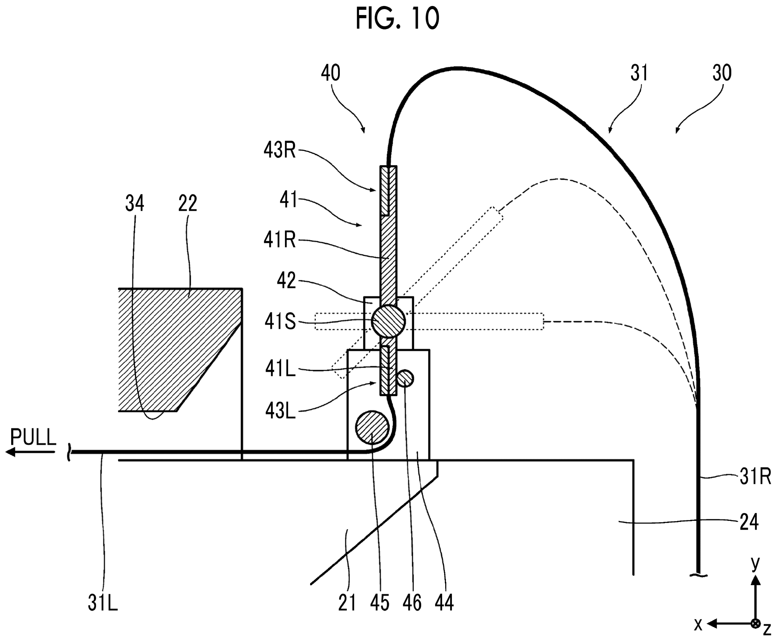

FIGS. 9 and 10 are views showing a first modification example of the belt elevating mechanism. FIG. 9 shows a state in which the right belt portion 31R is hanging down (a lowered state), and FIG. 10 shows a state in which the right belt portion 31R is pulled up (an elevated state).

In the belt elevating mechanism 40 of the present example, the configuration of rotating the rotating body 41 is different from that of the belt elevating mechanism 40 of the above-described embodiment. As shown in FIGS. 9 and 10, the rotating body 41 has a configuration in which the first arm portion 41R and the second arm portion 41L extend in opposite directions from the rotation shaft 41S (a configuration in which the second arm portion 41L extends in a direction opposite to the first arm portion 41R). In other words, the first arm portion 41R and the second arm portion 41L form an angle of 180° and are disposed in a plane with the rotation shaft 41S interposed therebetween (disposed in an I-shape in a cross section orthogonal to the rotation shaft 41S). The first arm portion 41R and the second arm portion 41L have different lengths in the radial direction of the rotation shaft 41S, that is, the first arm portion 41R has a length longer than a length of the second arm portion 41L, which is the same as the rotating body of the above-described embodiment.

The right belt portion 31R is connected to a distal end portion of the first arm portion 41R via the first connecting portion 43R provided on the distal end portion of the first arm portion 41R.

The left belt portion 31L is connected to a distal end portion of the second arm portion 41L via the second connecting portion 43L provided on the distal end portion of the second arm portion 41L.

Both ends of the rotation shaft 41S are rotatably supported by a pair of bearing portions 42 (only one side is shown in FIGS. 9 and 10).

The pair of bearing portions 42 are disposed on the top plate 21 via a pair of brackets 44 (only one side is shown in FIGS. 9 and 10). A guide bar 45 and a stopper bar 46 are disposed between the pair of brackets 44 with both ends thereof supported by the brackets 44.

The guide bar 45 is a guide member to which the left belt portion 31L is hooked and that guides a running of the left belt portion 31L. By hooking the left belt portion 31L to the guide bar 45, the first arm portion 41R rotates in a direction (counterclockwise direction in FIGS. 9 and 10) in which the first arm portion 41R stands by the pulling operation of the left belt portion 31L. Therefore, the guide bar 45 is disposed at a position at which the first arm portion 41R can stand in relation to the second arm portion 41L.

The stopper bar 46 is a restricting member that restricts the rotation range of the rotating body 41. As shown in FIG. 10, the stopper bar 46 abuts on the second arm portion 41L at a position at which the first arm portion 41R stands substantially vertically and restricts the rotation of the rotating body 41 (rotation in a counterclockwise direction in FIGS. 9 and 10). As a result, the rotating body 41 can be prevented from rotating excessively and colliding with the subject or the top plate 21. The rotation of the rotating body 41 in the reverse direction (rotation in a clockwise direction in FIGS. 9 and 10) is also restricted by the stopper bar 46. In this case, the first arm portion 41R abuts on the stopper bar 46 at a predetermined rotation position, and the rotation is restricted.

According to the belt elevating mechanism 40 of the present example configured as described above, the first arm portion 41R rotates in a direction (counterclockwise direction in FIGS. 9 and 10) in which the first arm portion 41R stands by the pulling operation of the left belt portion 31L, and as shown in FIG. 10, the right belt portion 31R is pulled up. On the other hand, the first arm portion 41R rotates in a direction (counterclockwise direction in FIGS. 9 and 10) in which the first arm portion 41R falls by the loosening operation of the left belt portion 31L (operation of releasing the pulling force), and as shown in FIG. 9, the right belt portion 31R is lowered.

As described above, the right belt portion 31R can also be elevated and lowered by the pulling operation and the loosening operation of the left belt portion 31L by the belt elevating mechanism 40 of the present example.

In a case of the belt elevating mechanism 40 of the present example, a space required for the rotation of the second arm portion 41L can be shifted downward as compared with the belt elevating mechanism 40 of the above-described embodiment. Therefore, the second arm portion 41L can be separated from the subject on the top plate 21.

### Second Modification Example

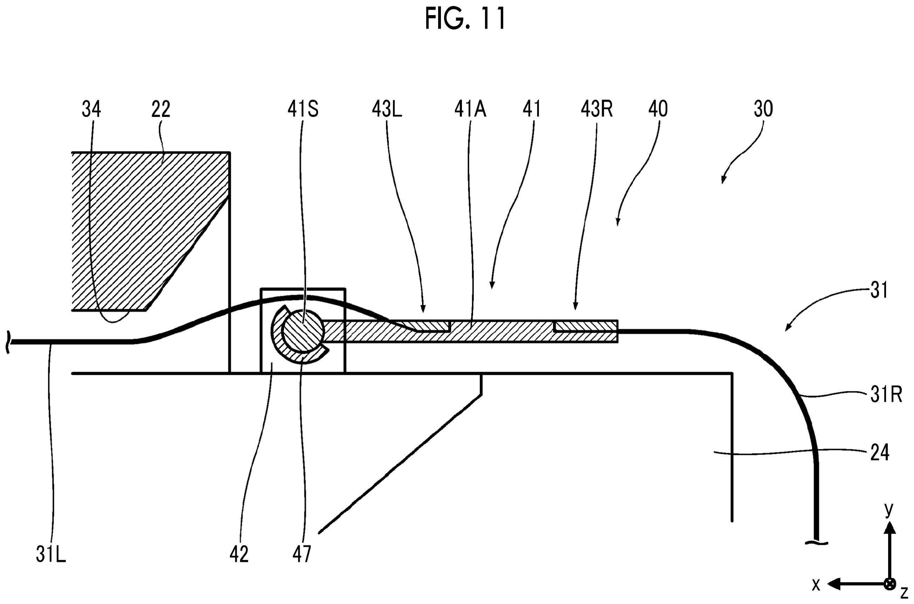

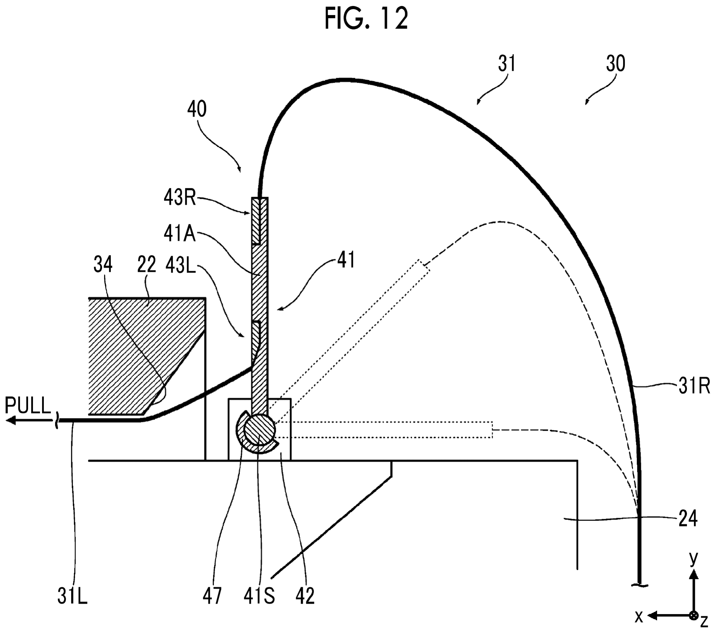

FIGS. 11 and 12 are views showing a second modification example of the belt elevating mechanism. FIG. 11 shows a state in which the right belt portion 31R is hanging down, and FIG. 12 shows a state in which the right belt portion 31R is pulled up.

The configuration of rotating the rotating body 41 in the belt elevating mechanism 40 of the present example is also different from that of the belt elevating mechanism 40 of the above-described embodiment. As shown in FIGS. 11 and 12, the rotating body 41 includes only one arm portion 41A. The arm portion 41A is configured of a rectangular plate-shaped member that extends in the radial direction from the rotation shaft 41S, and has the first connecting portion 43R on a distal end side and the second connecting portion 43L on a base end side. The right belt portion 31R is connected to the first connecting portion 43R, and the left belt portion 31L is connected to the second connecting portion 43L.

Both ends of the rotation shaft 41S are rotatably supported by the pair of bearing portions 42 (only one side is shown in FIGS. 11 and 12).

A rotation range restriction pipe 47 is disposed between the pair of bearing portions 42 with both ends thereof supported by the bearing portions 42. The rotation range restriction pipe 47 has a cross-sectional C-shaped shape and is disposed on an outer periphery of the rotation shaft 41S. The arm portion 41A swings within the range of the opening of the rotation range restriction pipe 47, so that the rotation range of the rotating body 41 is restricted. In the present example, the rotation range restriction pipe 47 is an example of a restricting member.

According to the belt elevating mechanism 40 of the present example configured as described above, the arm portion 41A rotates in a direction (counterclockwise direction in FIGS. 11 and 12) in which the arm portion 41A stands by the pulling operation of the left belt portion 31L, and as shown in FIG. 12, the right belt portion 31R is pulled up. On the other hand, the arm portion 41A rotates in a direction (counterclockwise direction in FIGS. 11 and 12) in which the arm portion 41A falls by the loosening operation of the left belt portion 31L, and as shown in FIG. 11, the right belt portion 31R is lowered.

As described above, the right belt portion 31R can also be elevated and lowered by the pulling operation and the loosening operation of the left belt portion 31L by the belt elevating mechanism 40 of the present example.

In a case of the belt elevating mechanism 40 of the present example, since the rotating body 41 can be configured by one arm portion 41A, the entire belt elevating mechanism 40 can be made compact, and the space required for the rotation operation can also be reduced.

### Third Modification Example

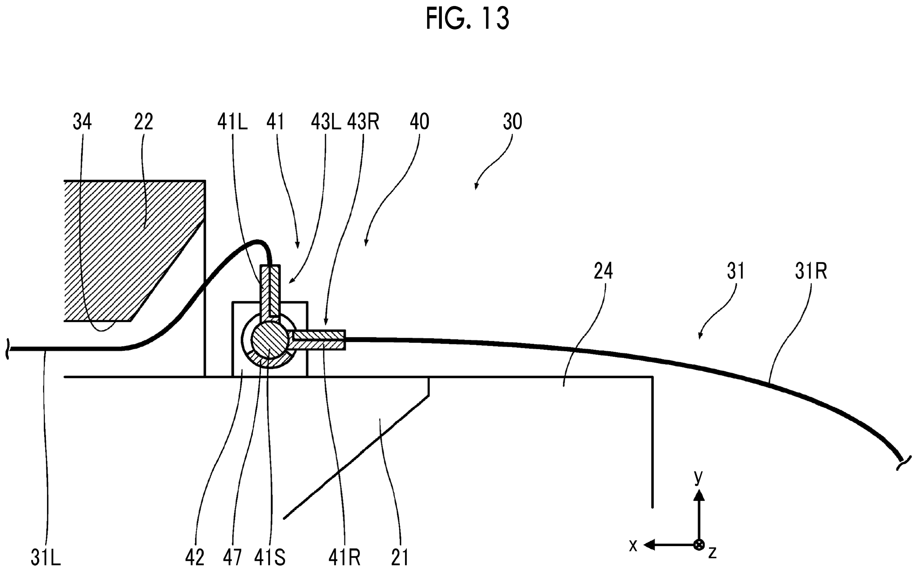

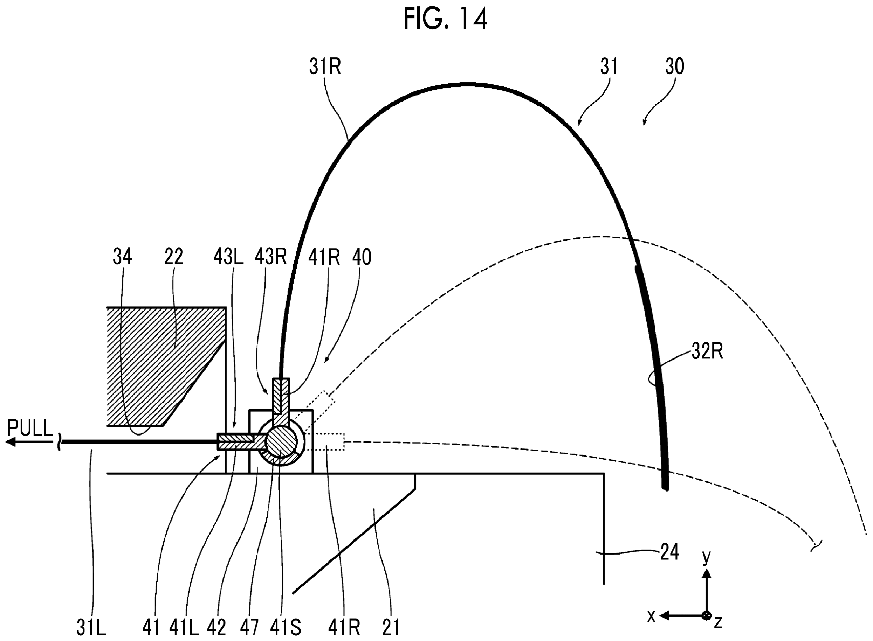

FIGS. 13 and 14 are views showing a third modification example of the belt elevating mechanism. FIG. 13 shows a state in which the right belt portion 31R is hanging down, and FIG. 14 shows a state in which the right belt portion 31R is pulled up.

In the belt elevating mechanism 40 of the present example, the right belt portion 31R is elevated and lowered by using the stiffness of the material of the right belt portion 31R. The stiffness which is also referred to as "body" or "hardness," is an index indicating resistance to bending (also referred to as rigidity). The higher the stiffness of the material (= the material with strong body), the greater the resistance to bending. Therefore, the self-supporting property is improved. That is, the right belt portion 31R is less likely to hang down in a vertical state.

As an example, as shown in FIG. 14, the right belt portion 31R is made of a material having a stiffness that the right belt portion 31R can be elevated to a predetermined height in a bent state in a case in which a lower end portion is held and the right belt portion 31R stands vertically. The predetermined height is a height (for example, a height that the right belt portion 31R pulled up can be easily retrieved by the technician from the side portion on the opposite side (left side) of the examination table 20 without excessively touching the subject P on the top plate 21) at which the right belt portion 31R pulled up can be easily retrieved.

It should be noted that, in a case in which the stiffness is too high (in a case in which the body is too strong), the right belt portion 31R is less likely to be wound around the subject in a case of fixing the subject, so that a material having an appropriate hardness is adopted. As an example, as the material of the right belt portion 31R, a flexible plastic material such as polyethylene terephthalate (PET) resin and a vinyl chloride resin can be adopted. For example, a vinyl chloride resin plate such as Kaidak (registered trademark) can be adopted.

In one right belt portion 31R, the stiffness may be changed between the base end portion side and the distal end portion side. That is, the stiffness on the base end portion side can be increased and the stiffness on the distal end portion side can be decreased. As a result, the distal end side can have flexibility while ensuring the self-supporting property.

It is preferable that the left belt portion 31L uses a material having a lower stiffness than the right belt portion 31R to be easily wound around the subject.

According to the belt elevating mechanism 40 of the present example, the right belt portion 31R can be elevated and lowered by using the stiffness of the material of the right belt portion 31R. As a result, the rotating body 41 can be made smaller. That is, since the length of the arm portion to which the right belt portion 31R is connected can be shortened, the entire apparatus can be made smaller.

### Fourth Modification Example

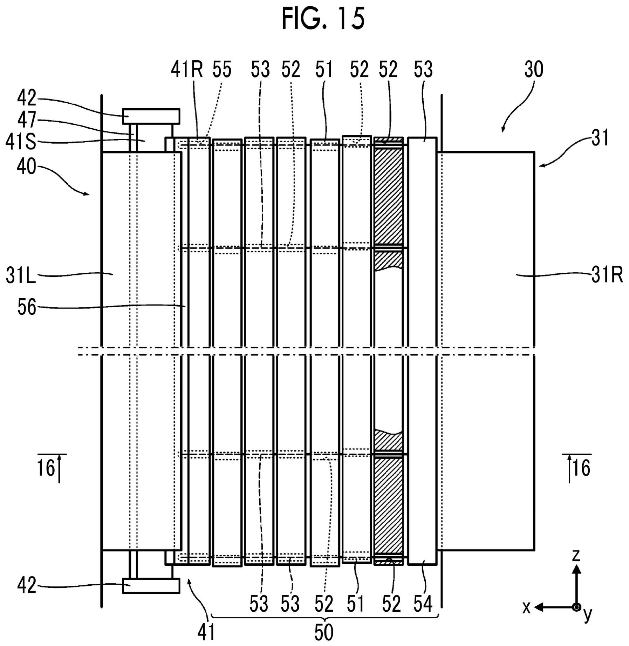

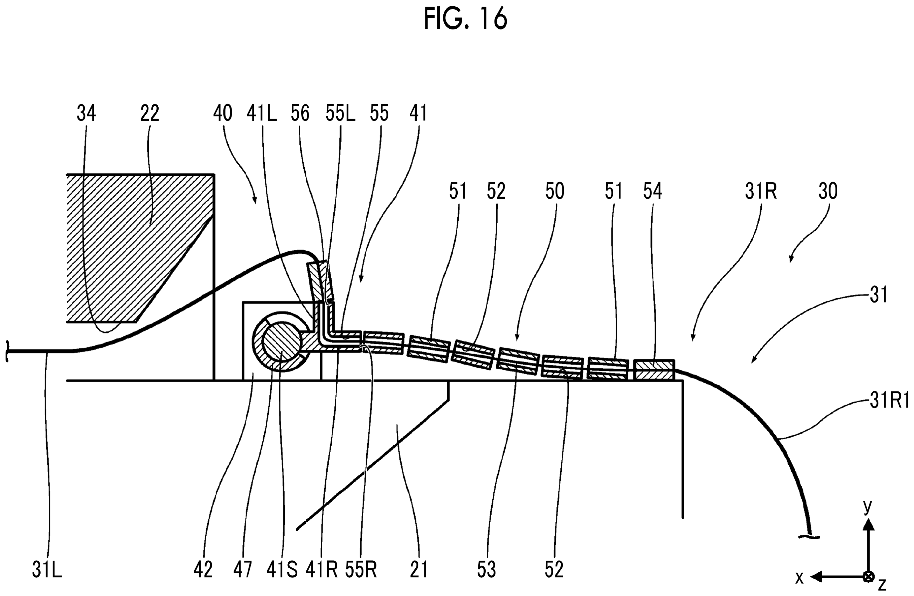

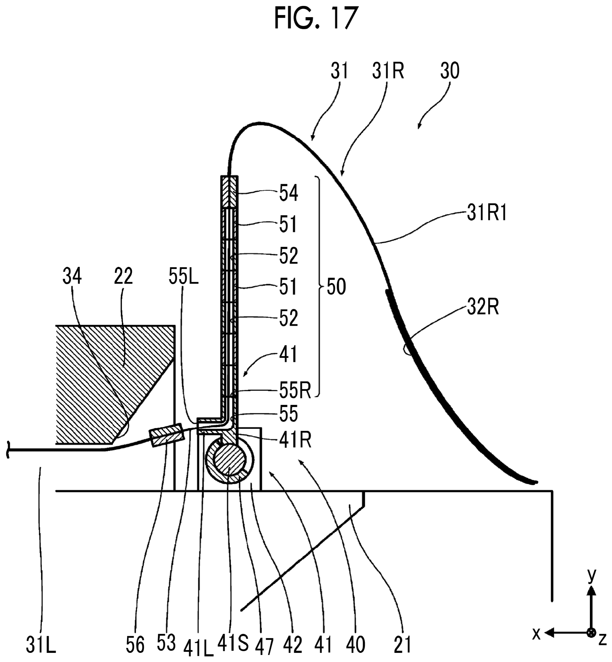

FIGS. 15 to 17 are views showing a fourth modification example of the belt elevating mechanism. FIG. 15 is a plan view of the right belt portion 31R including the belt elevating mechanism 40, and FIG. 16 is a cross-sectional view taken along a line 16-16 of FIG. 15. FIGS. 15 and 16 show a state in which the right belt portion 31R is hanging down, and FIG. 17 shows a state in which the right belt portion 31R is pulled up.

In the belt elevating mechanism 40 of the present example, the right belt portion 31R has a flexibility-variable portion at a base end portion. The flexibility of the flexibility-variable portion changes in conjunction with the pulling operation and the loosening operation of the left belt portion 31L. Specifically, the flexibility-variable portion is rigid in a planar shape by the pulling operation of the left belt portion 31L and is flexible so as to be bendable by the loosening operation of the left belt portion 31L.

As shown in FIGS. 15 to 17, the flexibility-variable portion 50 is configured by connecting a plurality of rectangular plate members 51 along the extending direction of the right belt portion 31R.

The plate member 51 is made of a rigid body and includes through-holes 52 at a plurality of locations in the longitudinal direction (z-axis direction of FIG. 15). A wire 53 is passed through the through-hole 52, and the plurality of plate members 51 are connected to each other (connected in a so-called bead chain manner). In the present example, the plurality of plate members 51 are an example of a plurality of members constituting the portion in which the flexibility changes.

A first retainer 54 is provided on a distal end portion of the flexibility-variable portion 50. The first retainer 54 has a rectangular plate shape and is disposed in a direction orthogonal to the extending direction (x-axis direction in FIG. 15) of the right belt portion 31R. A sheet-shaped portion 31R1 on the distal end side of the right belt portion 31R is fixed to the first retainer 54. In addition, one end of the wire 53 passed through the through-hole 52 of each plate member 51 is fixed to the first retainer 54.

The rotating body 41 includes the rotation shaft 41S, the first arm portion 41R that extends in the radial direction from the rotation shaft 41S, and the second arm portion 41L that extends in a direction orthogonal to the first arm portion 41R from the first arm portion 41R.

The rotating body 41 includes a plurality of guide holes 55 at a plurality of locations corresponding to the plurality of wires 53 extending from the first retainer 54. The guide hole 55 is provided in the rotating body 41 as a passage connecting an opening portion 55R provided on a distal end surface of the first arm portion 41R and an opening portion 55L provided on a distal end surface of the second arm portion 41L. The wire 53 extending from the first retainer 54 is introduced into the opening portion 55R at the distal end of the first arm portion 41R through the through-hole 52 of each plate member 51 and is pulled out from the opening portion 55L of the second arm portion 41L through the guide hole 55. In the present example, the distal end portion of the first arm portion 41R is an example of the first connecting portion.

The wire 53 pulled out from the opening portion 55L at the distal end of the second arm portion 41L has an end portion fixed to a second retainer 56. The second retainer 56 has a rectangular plate shape and is disposed in a direction orthogonal to the extending direction (x-axis direction of FIG. 15) of the left belt portion 31L. The base end portion of the left belt portion 31L is fixed to the second retainer 56. In the present example, the distal end portion of the second arm portion 41L is an example of a second connecting portion.

According to the belt elevating mechanism 40 of the present example configured as described above, in a case in which the left belt portion 31L is pulled, the wire 53 is pulled via the second retainer 56. In a case in which the wire 53 is pulled, the first retainer 54 connected to the distal end of the wire 53 is pulled toward the first arm portion 41R of the rotating body 41. As a result, the plate member 51 constituting the flexibility-variable portion 50 is interposed between the first retainer 54 and the first arm portion 41R and is solidified in a flat plate shape. That is, the plate members 51 adjacent to each other are joined to each other and are integrated in a flat plate shape.

In addition, in a case in which the left belt portion 31L is pulled, the rotating body 41 rotates, and the first arm portion 41R stands. As a result, as shown in FIG. 17, the right belt portion 31R is pulled up. As a result, the technician can retrieve the right belt portion 31R while being positioned on the left side (left belt portion 31L side) of the examination table 20.

In a case in which the pulling force of the left belt portion 31L is released and the left belt portion 31L is loosened, the wire 53 is also loosened. As a result, the clamping by the first retainer 54 and the first arm portion 41R is released, and the flexibility-variable portion 50 of the right belt portion 31R is flexible. That is, the adjacent plate members 51 are separated, and the flexibility-variable portion 50 are flexible so as to be bendable.

As described above, in the belt elevating mechanism 40 of the present example, the right belt portion 31R can also be elevated and lowered by the pulling operation and the loosening operation of the left belt portion 31L.

It should be noted that, in the present example, only a part (base end portion) of the right belt portion 31R is configured by the flexibility-variable portion, but the entire right belt portion 31R can also be configured by the flexibility-variable portion.

### Fifth Modification Example

The belt elevating mechanism may be configured to elevate and lower the right belt portion 31R by the pulling operation and the loosening operation of the left belt portion 31L. Therefore, in addition to the mechanism that elevates and lowers the rotating body, a link mechanism, a cam mechanism, or the like may be used.

### Modification Example of Fixing Belt

The right belt portion 31R and the left belt portion 31L do not necessarily need to be configured of a belt-shaped sheet as a whole. For example, a portion of the left belt portion 31L that passes through the space 34 can also be configured of a plurality of strings, wires, or the like.

In addition, in the above-described embodiment, the hook-and-loop fasteners 32R and 32L are used to join the right belt portion 31R and the left belt portion 31L, but the means for joining the right belt portion 31R and the left belt portion 31L is not limited thereto. Additionally, it may also be configured to join using, for example, point fasteners (such as buttons and hooks), line fasteners, or the like.

### Modification Example of Space for Passing Left Belt Portion

In the above-described embodiment, the space 34 through which the left belt portion 31L passes is provided in the mattress 22, but may be provided on the top plate 21. Alternatively, a configuration in which the space 34 is formed between the top plate 21 and the mattress 22 by placing the mattress 22 on the top plate 21 may be adopted.

### Second Embodiment

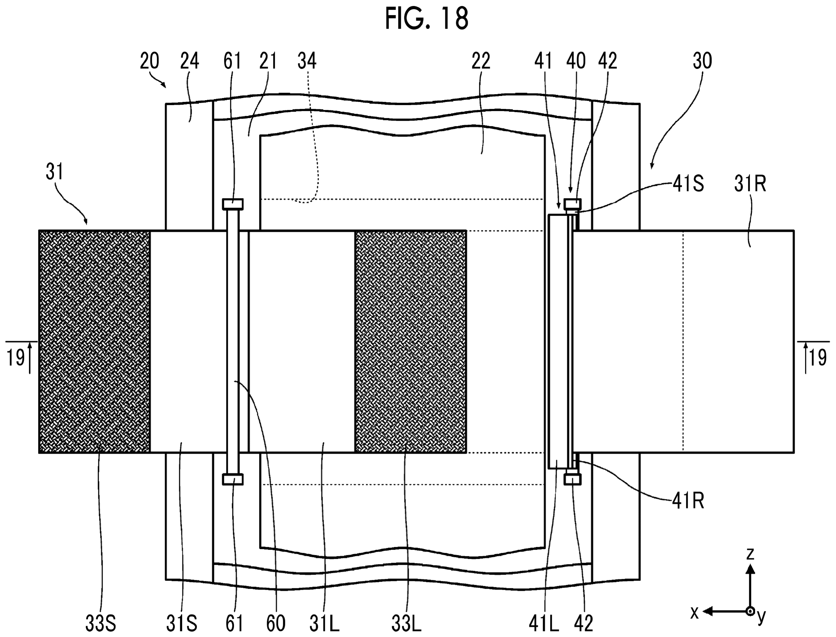

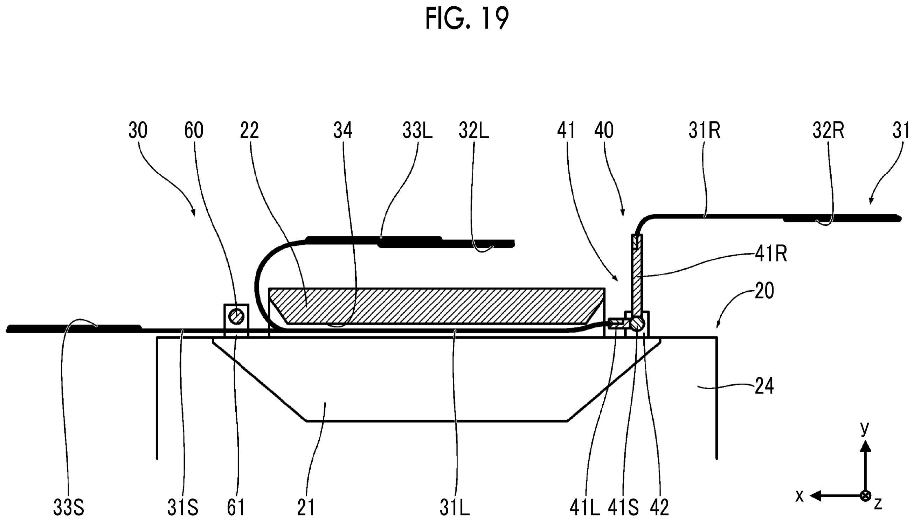

FIG. 18 is a plan view of a second embodiment of the fixing tool. FIG. 19 is a cross-sectional view taken along a line 19-19 of FIG. 18.

In the fixing tool 30 of the present embodiment, the configuration of the fixing belt 31 is different from that of the fixing tool 30 of the first embodiment. Therefore, here, only the difference from the fixing tool 30 of the first embodiment will be described.

As shown in FIGS. 18 and 19, the fixing belt 31 of the present embodiment further includes an auxiliary belt portion 31S.

The auxiliary belt portion 31S has a disposition configuration in which the auxiliary belt portion 31S branches from the left belt portion 31L and extends from a side portion on the left side of the top plate 21 in the traveling direction of the top plate 21 to the lateral direction of the top plate 21 together with the left belt portion 31L. In addition, the auxiliary belt portion 31S is configured of a flexible belt-shaped sheet, as in the right belt portion 31R and the left belt portion 31L. In the present embodiment, the auxiliary belt portion 31S is an example of a third belt portion.

The left belt portion 31L and the auxiliary belt portion 31S have a configuration in which the auxiliary belt portion 31S is disposed to overlap the left belt portion 31L on the outer side in a case in which the left belt portion 31L and the auxiliary belt portion 31S are wound around the subject.

The hook-and-loop fasteners 33L and 33S are provided on overlapping surfaces of the distal end portions of the left belt portion 31L and the auxiliary belt portion 31S when distal end portions of the left belt portion 31L and the auxiliary belt portion 31S are wound around the subject (mutually facing surfaces of the right belt portion 31R and the left belt portion 31L when the distal end portions of the left belt portion 31L and the auxiliary belt portion 31S are overlapped), and the distal end portions of the left belt portion 31L and the auxiliary belt portion 31S are joined to each other. As described above, in the present embodiment, the auxiliary belt portion 31S is disposed to overlap the left belt portion 31L on the outer side in a case in which the auxiliary belt portion 31S is wound around the subject. The left belt portion 31L includes the hook-and-loop fastener 33L on a surface that is disposed on the outer side (upper side) in a case in which the left belt portion 31L is wound around the subject P. On the other hand, the auxiliary belt portion 31S includes the hook-and-loop fastener 33S on a surface that is disposed on the inner side (lower side) in a case of being wound around the subject P. As an example, in the present embodiment, the hook-and-loop fastener 33L on the left belt portion 31L side has a configuration in which the hook-and-loop fastener 33L is hooked, and the hook-and-loop fastener 33S on the auxiliary belt portion 31S side has a configuration in which the hook-and-loop fastener 33S is hooked.

An auxiliary bar 60 is provided on the top plate 21 at a position facing the belt elevating mechanism 40 across the mattress 22. The auxiliary bar 60 has a round bar shape and is disposed along the longitudinal direction (z-axis direction of FIG. 18) of the top plate 21. In addition, both ends of the auxiliary bar 60 are supported by brackets 61 and are disposed at a position at a predetermined height from the upper surface of the top plate 21.

The auxiliary belt portion 31S is pulled out to the side portion on the left side of the examination table 20 through the lower side of the auxiliary bar 60 (between the upper surface of the top plate 21 and the auxiliary bar 60). On the other hand, the left belt portion 31L is pulled out to the side portion on the left side of the examination table 20 through the upper side of the auxiliary bar 60 (between the auxiliary bar 60 and the mattress 22).

Therefore, in a case in which the auxiliary belt portion 31S is wound around the subject, the auxiliary belt portion 31S is hooked to the auxiliary bar 60 and is wound around the subject. On the other hand, the left belt portion 31L is wound around the subject without passing through the auxiliary bar 60. In the present embodiment, the auxiliary bar 60 is an example of an engagement portion that is hooked and engaged with the auxiliary belt portion 31S.

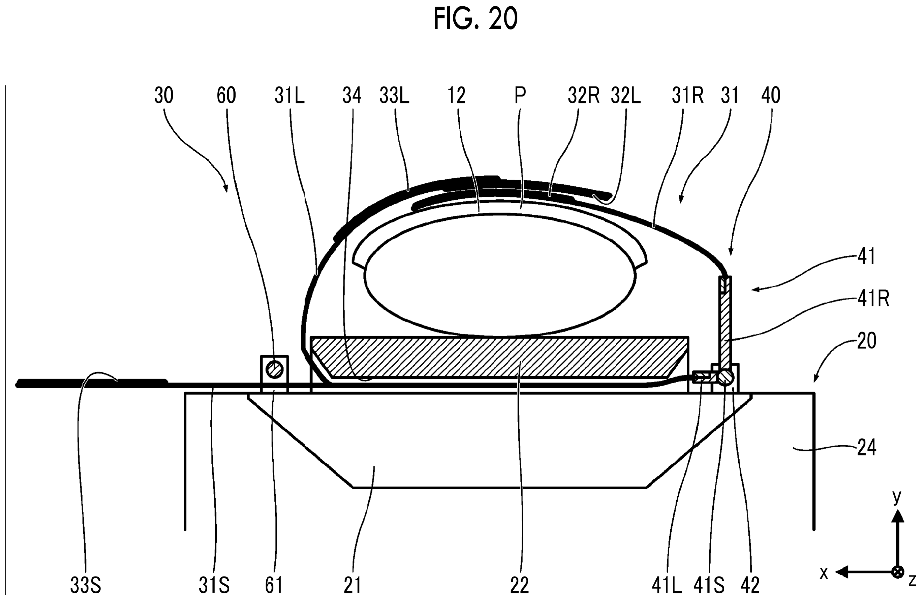

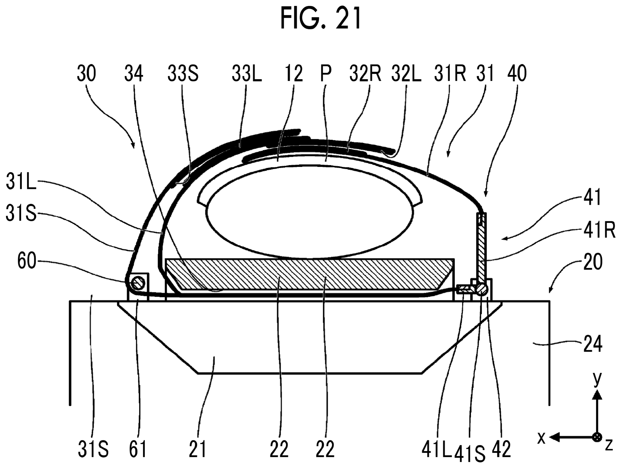

Next, a method of fixing the subject P on the top plate 21 using the fixing tool 30 of the present embodiment will be described. FIGS. 20 and 21 are explanatory views of a method of fixing the subject using the fixing tool.

First, the subject P is placed on the mattress 22 of the top plate 21, and the receive coil 12 is set at the imaging target portion. After the receive coil 12 is set, the left belt portion 31L and the auxiliary belt portion 31S are pulled, and the right belt portion 31R is pulled up and retrieved.

The retrieved right belt portion 31R and the left belt portion 31L are wound around the subject P on the top plate 21, and the right belt portion 31R and the left belt portion 31L are joined to each other. As shown in FIG. 20, the right belt portion 31R and the left belt portion 31L are overlapped with each other, and the hook-and-loop fastener 32R on the right belt portion 31R side and the hook-and-loop fastener 32L on the left belt portion 31L side are adhered to each other to be joined to each other.

After the right belt portion 31R and the left belt portion 31L are joined to each other, as shown in FIG. 21, the auxiliary belt portion 31S is wound around the subject P via the auxiliary bar 60 and is joined to the left belt portion 31L. The auxiliary belt portion 31S is overlapped on the left belt portion 31L, and the hook-and-loop fastener 33S is adhered to the hook-and-loop fastener 33L on the left belt portion 31L side to be joined to the left belt portion 31L.

As described above, according to the fixing tool 30 of the present embodiment, the fixing belt 31 includes the auxiliary belt portion 31S. As a result, so-called additional tightening can be performed, and the subject P on the top plate 21 can be more reliably fixed. That is, in a case in which the fixing using only the left belt portion 31L is insufficient, the additional tightening is performed using the auxiliary belt portion 31S, so that the subject P on the top plate 21 can be more reliably fixed.

It should be noted that, in the present embodiment, the auxiliary belt portion 31S is joined to the left belt portion 31L, but may be joined to the right belt portion 31R. In addition, the auxiliary belt portion 31S may be configured to be joinable to both the right belt portion 31R and the left belt portion 31L.

In addition, in the above-described embodiment, the hook-and-loop fasteners 32R and 32L are used to join the right belt portion 31R and the left belt portion 31L, but the means for joining the right belt portion 31R and the left belt portion 31L is not limited thereto. Additionally, it may also be configured to join using, for example, point fasteners (such as buttons and hooks), line fasteners, or the like. The same applies to the joining of the auxiliary belt portion 31S and the left belt portion 31L.

In a case in which a hook-shaped fastener (so-called hook) is used to join the right belt portion 31R and the left belt portion 31L, the fastener is pulled by the joining of the auxiliary belt portion 31S, so that the right belt portion 31R and the left belt portion 31L can be more firmly engaged. That is, since the retainer is pulled by the pulling operation of the auxiliary belt portion 31S for the additional tightening, the looseness can be suppressed, and the right belt portion 31R and the left belt portion 31L can be more firmly joined to each other. In this case, it is preferable to use a hook-and-loop fastener that can adjust a joining position for joining the auxiliary belt portion 31S.

In addition, in the present embodiment, the round bar-shaped auxiliary bar 60 is used as the engagement portion of the auxiliary belt portion 31S, but the form of the engagement portion is not limited thereto. In addition, for example, a configuration in which an auxiliary belt portion 31S is engaged with a square can or the like provided on the top plate 21 can also be adopted.

In addition, various forms can be adopted for the belt elevating mechanism 40 including the modification examples described in the above-described embodiment.

### Third Embodiment

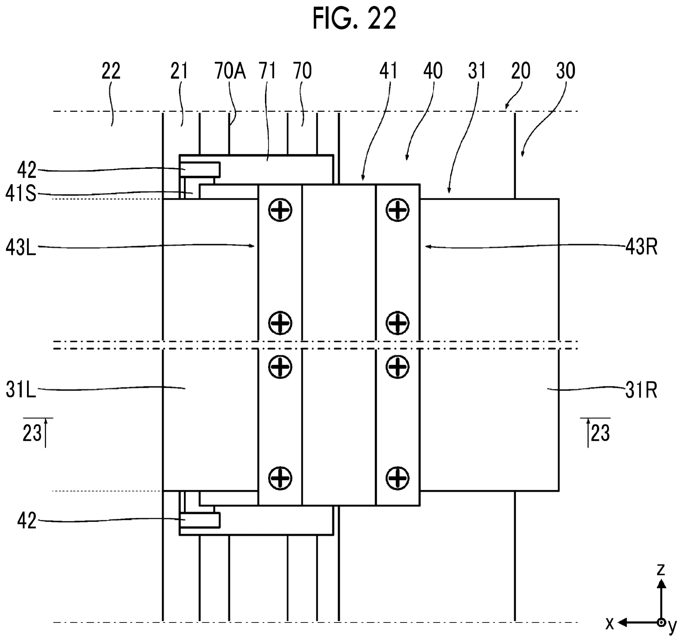

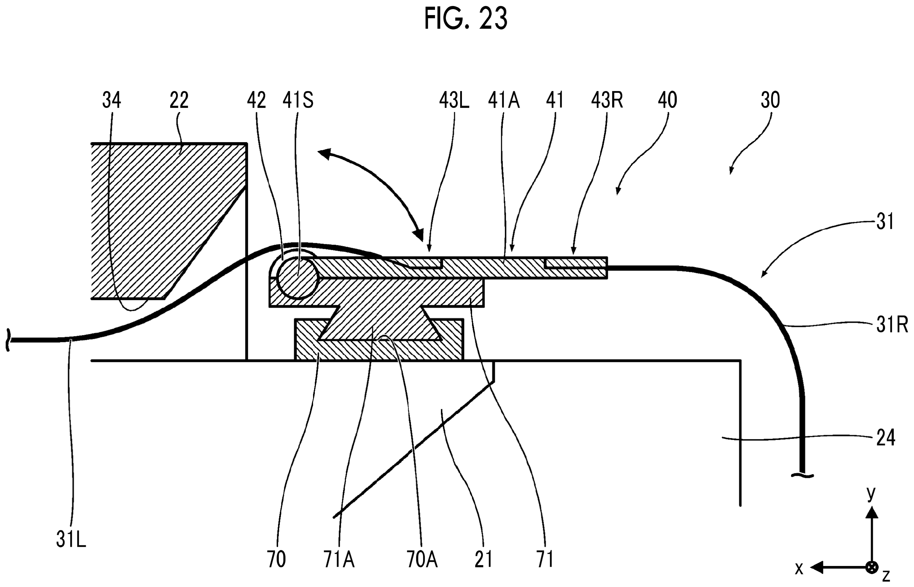

FIG. 22 is a plan view of a third embodiment of the fixing tool. FIG. 23 is a cross-sectional view taken along a line 23-23 of FIG. 22.

In the fixing tool 30 of the present embodiment, the belt elevating mechanism 40 is provided to be movable along the longitudinal direction (z-axis direction of FIG. 22) of the top plate 21, and the position at which the subject P is fixed can be changed.

As shown in FIGS. 22 and 23, a guide rail 70 and a slider 71 that slides along the guide rail 70 are provided on the top plate 21.

The guide rail 70 is disposed along an edge of the top plate 21 on one side (the disposition side of the belt elevating mechanism 40). Therefore, the guide rail 70 is disposed along the longitudinal direction of the top plate 21. The guide rail 70 includes a guide groove 70A having a cross-sectional trapezoidal shape (so-called key groove shape).

The slider 71 has a sliding member 71A having a cross-sectional trapezoidal shape on a back surface, and the sliding member 71A slides and is engaged with the guide groove 70A of the guide rail 70 to be slidably supported on the guide rail 70. The slider 71 includes a lock mechanism (not shown) and can be locked at any position on the guide rail 70.

The belt elevating mechanism 40 is installed on the slider 71. As a result, the belt elevating mechanism 40 is provided to be movable along the longitudinal direction of the top plate 21 via the slider 71.

FIGS. 22 and 23 show an example of a case in which the rotating body 41 includes only one arm portion 41A (see the second modification example of the belt elevating mechanism 40). The belt elevating mechanism mounted on the slider 71 is not limited thereto, and other configurations can also be adopted. In a case in which the belt elevating mechanism 40 has the configuration shown in FIGS. 22 and 23, the arm portion 41A rotates (swings) in a butterfly shape with respect to the slider 71.

The space 34 through which the left belt portion 31L passes is provided on the mattress 22 in accordance with the movable range of the belt elevating mechanism 40.

According to the fixing tool 30 of the present embodiment configured as described above, the installation position of the belt elevating mechanism 40 can be adjusted along the longitudinal direction of the top plate 21. As a result, the position at which the subject is fixed by the fixing tool 30 can be adjusted. That is, the fixed position can be adjusted along the body axis direction of the subject.

### Other Embodiments

In the above-described embodiment, the configuration in which the mattress 22 is provided on the top plate 21 is adopted, but a configuration in which the mattress 22 is not provided can also be adopted. In this case, the space through which the belt passes is provided on the top plate 21.

In addition, in the above-described embodiment, the case in which one fixing tool 30 is provided on one top plate 21 is described as an example, but a configuration in which a plurality of fixing tools 30 are provided on one top plate 21 can also be adopted. That is, a configuration in which the fixing can be performed at a plurality of locations can also be adopted.

In addition, in the above embodiment, a case in which the present invention is applied to the MRI apparatus has been described as an example, but the application of the present invention is not limited to this. The present invention can be applied to a medical apparatus comprising an examination table (medical examination table), particularly, a medical image diagnostic apparatus (an apparatus that images a medical image of a subject placed on a medical examination table) such as an X-ray diagnostic apparatus, an X-ray CT apparatus, a PET apparatus, and an SPECT apparatus.

In addition, the configurations of each of the above-described embodiments can be appropriately combined including the modification examples.

In the present specification, the meanings of the terms "the same" and "identical" include not only a meaning of "completely identical" but also a meaning of "substantially the same" including an error allowed in design and manufacturing. In addition, in the present specification, a meaning of the term "orthogonal" includes not only a meaning of "perfectly orthogonal" but also a meaning of "substantially orthogonal" including an error allowed in design and manufacturing.

### Explanation of References

1: MRI apparatus
10: magnet
11: imaging space
12: receive coil
20: examination table
21: top plate
22: mattress
23: base
24: top-plate support portion
30: fixing tool
31: fixing belt
31L: left belt portion
31R: right belt portion
31R1: portion
31S: auxiliary belt portion
32L: hook-and-loop fastener
32R: hook-and-loop fastener
33L: hook-and-loop fastener
33S: hook-and-loop fastener
34: space
40: belt elevating mechanism
41: rotating body
41A: arm portion
41L: second arm portion
41R: first arm portion
41S: rotation shaft
42: bearing portion
43L: second connecting portion
43L1: clamping member
43R: first connecting portion
43R1: clamping member
44: bracket
45: guide bar
46: stopper bar
47: rotation range restriction pipe
50: flexibility-variable portion
38: plate member
52: through-hole
53: wire
54: first retainer
55: guide hole
55L: opening portion of guide hole
55R: opening portion of guide hole
56: second retainer
60: auxiliary bar
61: bracket
70: guide rail
70A: guide groove
71: slider
71A: sliding member
CCW: rotation direction (counterclockwise) of rotating body
CW: rotation direction (clockwise) of rotating body
P: subject

## Claims

1. A medical examination table (20) comprising:
a placement portion on which a subject (P) is placed;
a fixing belt (31) that includes a first belt portion extending in a lateral direction of the placement portion from a first side portion that is a side portion on one side of the placement portion in the lateral direction, and a second belt portion extending in the lateral direction of the placement portion from a second side portion that is a side portion on the other side of the placement portion in the lateral direction of the placement portion, and that fixes the subject (P) placed on the placement portion by joining the first belt portion and the second belt portion; and
an elevating mechanism that is provided on a first side portion side of the placement portion, that includes a first connecting portion (43R) to which the first belt portion is connected, and a second connecting portion (43L) to which the second belt portion is connected, and that elevates at least a part of the first belt portion by pulling operation of the second belt portion and lowers the at least part of the first belt portion by loosening operation of the second belt portion.

2. The medical examination table (20) according to claim 1,
wherein the elevating mechanism includes a rotating member that rotates about an axis extending in a longitudinal direction of the placement portion,
the first connecting portion (43R) and the second connecting portion (43L) are provided in the rotating member, and
the rotating member is rotated by the pulling operation and the loosening operation of the second belt portion to elevate and lower the at least part of the first belt portion.

3. The medical examination table (20) according to claim 2,
wherein the rotating member includes
a shaft portion,
a first arm portion (41R) that extends in a radial direction from the shaft portion, and
a second arm portion (41L) that extends in a direction intersecting the first arm portion (41R) from the shaft portion or the first arm portion (41R),
the first connecting portion (43R) is provided in the first arm portion (41R), and
the second connecting portion (43L) is provided in the second arm portion (41L).

4. The medical examination table (20) according to claim 3,
wherein the first connecting portion (43R) is provided on a distal end portion of the first arm portion (41R),
the second connecting portion (43L) is provided on a distal end portion of the second arm portion (41L), and
a length of the first arm portion (41R) is longer than a length of the second arm portion (41L).

5. The medical examination table (20) according to claim 2,
wherein the rotating member includes
a shaft portion,
a first arm portion (41R) that extends in a radial direction from the shaft portion, and
a second arm portion (41L) that extends in a direction opposite to the first arm portion (41R) from the shaft portion,
the first connecting portion (43R) is provided in the first arm portion (41R),
the second connecting portion (43L) is provided in the second arm portion (41L), and
the elevating mechanism further includes a guide member that guides the second belt portion in a direction in which the rotating member rotates in a case in which the second belt portion is pulled.

6. The medical examination table (20) according to claim 2,
wherein the rotating member includes
a shaft portion and
an arm portion (41A) that extends in a radial direction from the shaft portion,
the first connecting portion (43R) is provided on a distal end side of the arm portion (41A), and
the second connecting portion (43L) is provided on a base end side of the arm portion (41A).

7. The medical examination table (20) according to claim 2,
wherein the first belt portion has a higher stiffness than the second belt portion.

8. The medical examination table (20) according to claim 2,
wherein the first belt portion has a portion (31R1) of which flexibility changes in conjunction with the pulling operation and the loosening operation of the second belt portion at least at a base end portion, and
the portion (31R1) is configured by connecting a plurality of members in an extending direction of the first belt portion, is rigid by joining the members adjacent to each other by the pulling operation of the second belt portion and is flexible by separating the members adjacent to each other by the loosening operation of the second belt portion.

9. The medical examination table (20) according to any one of claims 1 to 8,
wherein the fixing belt (31) further includes a third belt portion that branches from the second belt portion, that extends from the second side portion of the placement portion in the lateral direction of the placement portion together with the second belt portion, and that is joinable to the first belt portion and/or the second belt portion, and
preferably, the medical examination table (20) further comprises an engagement portion that is provided on a second side portion side of the placement portion and on which the third belt portion is hooked and engaged.

10. The medical examination table (20) according to claim 9,
wherein the second belt portion is joined to the first belt portion via a hook-shaped retainer, and
the third belt portion is joined to the first belt portion and/or the second belt portion via a hook-and-loop fastener (32L, 32R, 33L, 33S).

11. The medical examination table (20) according to any one of claims 1 to 10,
wherein the elevating mechanism is movable in a longitudinal direction of the placement portion.

12. The medical examination table (20) according to any one of claims 2 to 8,
wherein the elevating mechanism further includes a restricting member that restricts a rotation range of the rotating member.

13. The medical examination table (20) according to any one of claims 1 to 12,
wherein the placement portion has a space (34) through which the second belt portion passes from the first side portion side to a second side portion side.

14. The medical examination table (20) according to claim 13,
wherein the placement portion includes
a top plate (21),
a mattress (22) provided on the top plate (21), and
the space (34) is provided on the top plate (21) or the mattress (22) or between the top plate (21) and the mattress (22).

15. A medical image diagnostic apparatus (1) comprising:
the medical examination table (20) according to any one of claims 1 to 14,
wherein the medical image diagnostic apparatus (1) captures a medical image of the subject (P) placed on the medical examination table (20).
